# EUROPÄISCHE PATENTSCHRIFT

(11) **EP 0 756 502 B1**
(45) Veröffentlichungstag und Bekanntmachung des Hinweises auf die Patenterteilung: **03.03.1999**
(21) Anmeldenummer: 95915870.0
(22) Anmeldetag: 12.04.1995
(51) Int. Cl.: A61M 16/00

(54) **BEATMUNGSGERÄT, INSBESONDERE ZUR BEHANDLUNG VON ATEMINSUFFIZIENZEN, SOWIE VERFAHREN ZU DESSEN BETRIEB**
RESPIRATOR, IN PARTICULAR FOR THE TREATMENT OF RESPIRATORY INSUFFICIENCY, AND A METHOD OF OPERATING SAID RESPIRATOR
RESPIRATEUR S'UTILISANT NOTAMMENT DANS LE TRAITEMENT DES INSUFFISANCES RESPIRATOIRES, ET SON PROCEDE DE FONCTIONNEMENT

(30) Priorität: 19.04.1994 DE 9406484 U
(43) Veröffentlichungstag der Anmeldung: 05.02.1997
(73) Patentinhaber: Bösherz, Jakob, 94065 Waldkirchen (DE); Bauer, Heribert, 94065 Waldkirchen (DE)
(72) Erfinder: Bösherz, Jakob, 94065 Waldkirchen (DE); Bauer, Heribert, 94065 Waldkirchen (DE)
(74) Vertreter: Brandl, Ferdinand Anton, Dipl.-Phys.
(86) Internationale Anmeldenummer: EP9501368
(87) Internationale Veröffentlichungsnummer: WO9528193

(56) Entgegenhaltungen:
- EP-A- 0 245 142
- EP-A- 0 413 127
- EP-A- 0 459 647
- WO-A-92/11054
- WO-A-93/00952
- WO-A-93/08857
- WO-A-93/21982
- FR-A- 2 407 020
- GB-A- 2 033 759
- US-A- 4 986 268
- US-A- 5 050 593

## Beschreibung

Die Erfindung betrifft ein Beatmungsgerät, sowie ein Verfahren zu dessen Betrieb.

Beatmungsgeräte üblicher Bauart weisen einen Preßgasanschluß, Magnetventile, Mikroprozessoren, eine Wasserpumpe, ein Venturi-Rohr, Sensoren und Alarmsysteme auf. Solche Beatmungsgeräte werden im Grund für Hochfrequenzbeatmung verwendet. Beispiele hierfür sind in der Literatur zu finden, z. B.: "*Lehrbuch der Anästhesiologie und Intensivmedizin*" Bd. 2, H. Benzer, H. Burchardi, R. Larsen, P.M. Suter 6. Aufl. S. 429. Damit ist aber eine Darstellung einer Vielzahl verschiedener Flow-Muster und Beatmungsformen nicht möglich, der Sauerstoffverbrauch ist hoch, die Durchführung von volumenkontrollierten Beatmungsformen ist erschwert.

Bekannt sind auch Beatmungsgeräte, die einen Preßgasanschluß, Stromwandler, Verstärker, einen Mikroprozessor und Beatmungsschläuche aufweisen. Solche Geräte werden zur Anwendung bekannter Standard-Flow-Muster verwendet. Ein Literaturbeispiel hierfür ist: "*Respiratoren in der klinischen Praxis*", 2. Aufl., M. Dittmann, S. 203.

Aus der US-A-4 986 268 ist ein Beatmungsgerät bekannt, bei dem im Beatmungskanal ein Sensor zur Messung der expiratorischen Sauerstoffkonzentration angeordnet ist.

Die US-A-5 050 593 offenbart ein Beatmungsgerät, von welchem die vorliegende Erfindung gemäß Anspruch 1 ausgeht. Dieses bekannte Beatmungsgerät verwendet ein Inspirationsventil mit einem starren äußeren Zylinder und einem elastischen inneren Zylinder und einem dazwischen liegenden Raum. Das Ventil ist im inspiratiorischen Kanal zwischen dem inspiratiorischen Ende eines Konnektors und dem inspiratorischen Schlauch eines Respirators angebracht. Bei Triggerversuchen des Patienten entsteht im Konnektor ein Unterdruck, der den inneren Zylinder zum Kollabieren bringt. Dabei kommt der Zylinder in Kontakt mit einer Scheibe und verschließt den inspiratorischen Kanal völlig. Im Falle der US-A-5 050 593 handelt es sich somit um einen Trigger-Mechanismus, also um eine Einrichtung, die die Inspirationsversuche eines Patienten erkennt, verstärkt und vom Respirator einen Zwangsatemzug auslöst. Das Inspirationsventil öffnet sich bei Triggerversuchen des Patienten und nach dem Erreichen des eingestellten endexpiratorischen Druckes im Konnektor und startet somit die Inspiration vom Respirator. Nach dem Erreichen des eingestellten Tidalvolumens oder Beatmungsdruckes schließt sich das Ventil und beendet somit die Inspiration. Da somit das Ventil nur zwischen zwei Endlagen (AUF/ZU) umsteuert, kann seine Querschnittsweite nicht steuerbar verändert werden. Zur Erzeugung von regelbaren Beatmungsdrücken, -flows und -widerständen ist diese Einrichtung nicht geeignet.

Schließlich ergeben sich bei den bekannten Beatmungsgeräten noch weitere Nachteile und Einschränkungen, die im praktischen Betrieb zum Teil ganz gravierend sein können: wie bereits erwähnt, sind die Versionen der Flow-Muster stark eingeschränkt bzw. begrenzt. Eine Durchführung von Hochfrequenzbeatmung und verschiedene Mischformen ist nicht möglich. Die Geräte können nicht fernbedient werden. Eine kontinuierliche endotracheale Medikamenteninstillation ist nicht vorgesehen.

Die bekannten Geräte haben auch ergonomische Nachteile, z. B. benötigen sie Demontage, Montage und Sterilisation der in- und exspiratorischen Kanäle und sie begrenzen den Zugang des Personals zum Patienten. Schließlich haben diese Geräte einen erheblichen Sauerstoffverbrauch.

Es ist daher Aufgabe der vorliegenden Erfindung, ein Beatmungsgerät bzw. Verfahren zu dessen Betrieb zur Verfügung zu stellen, das beliebige Beatmungsformen und Flowmuster darstellen kann.

Die Lösung dieser Aufgabe erfolgt bezüglich des Beatmungsgerätes durch die Merkmale des Anspruchs 1 und bezüglich der Verfahren insbesondere zu dessen Betrieb durch die Merkmale des Anspruches 17.

Ein Beatmungsgerät gemäß der vorliegenden Erfindung weist somit einen mit dem Patienten verbindbaren Beatmungskanal mit einem proximalen und einem distalen Ende, Hochdruckgasanschlüsse für Beatmungsgase, eine Steuereinrichtung zum Festlegen von Beatmungsparametern und eine Einrichtung zur Dosierung der Beatmungsgase auf und ist dadurch gekennzeichnet, daß der Beatmungskanal in Form eines Venturi-Rohres eine Variodüse mit einstellbarem Querschnitt flußabwärts einer Injektordüse, eine Varioblende mit einstellbarem Querschnitt flußaufwärts der Injektordüse, welche die Menge der eingesaugten Raumluft dosiert begrenzt und einen endexpiratorischen Sauerstoffkonzentrationssensor zur Oxygenationskontrolle aufweist, wobei die Ausgangssignale des Sauerstoffkonzentrationssensors zur Regelung der variablen Querschnitte der Varioblende und zur Einstellung des Querschnittes der Variodüse verwendet werden.

Bei einem erfindungsgemäßen Verfahren zum Betrieb eines Beatmungsgerätes werden zur Dosierung des Sauerstoffes in den Beatmungsgase die im Anspruch 17 angegebenen Schritte unternommen.

Vorteilhafte Ausgestaltungsformen des Beatmungsgerätes bzw. des Verfahrens zu dessen Betrieb sind Gegenstand der diesbezüglichen Unteransprüche.

So weist die Variodüse bevorzugt einen starren äußeren Zylinder und einen inneren elastischen und zur Mitte bewegbaren Zylinder mit einem dazwischenliegenden Raum und ein Mikroventuri-Rohr auf, das am äußeren starren Zylinder angeordnet ist.

Die Variodüse ist bevorzugt mit einer Steuereinrichtung zum Einbringen eines Druckes in den Raum zwischen den Zylindern versehen, welche das Mikroventuri-Rohr, einen mikroelektropneumatischen Umwandler und einen Computer aufweist.

Der Raum zwischen dem äußeren und dem inneren Zylinder der Variodüse steht bevorzugt über das Mikroventuri-Rohr mit der Umgebung in Verbindung.

Der Raum zwischen dem äußeren und inneren Zylinder der Variodüse ist bevorzugt über einen mikroelektropneumatischen Wandler mit dem Hochdruckgasanschluß verbunden.

Die Dosiereinrichtung kann vorteilhafterweise einen elektropneumatischen Umwandler für hohe Drücke aufweisen.

Die Varioblende weist bevorzugt einen starren äußeren Zylinder, einen inneren elastischen und zur Mitte bewegbaren Zylinder und einen dazwischen liegenden Raum auf, wobei die Verengungen des Innenlumens auch völlig verschließbar ist, sowie ein Mikroventuri-Rohr, das am äußeren starren Zylinder angeordnet ist.

Die Varioblende ist bevorzugtmit einer Steuereinrichtung mit dem Mikroventuri-Rohr, einem mikroelektropneumatischen Umwandler und dem Computer zum Einbringen eines Druckes in den Raum zwischen den Zylindern verbunden.

Das Venturi-Rohr weist bevorzugt einen Geräuschesensor auf.

Das Beatmungsgerät weist weiterhin bevorzugt im Hochdruckkanal oberhalb der Injektordüse einen Blitzwasserverdampfer auf.

Der Blitzwasserverdampfer weist bevorzugt Fluidverbindungen zu einer Wasserpumpe, pneumatische Verbindungen zur Injektordüse und zum elektropneumatischen Umwandler, sowie elektronische Verbindungen zum Computer auf.

Das distale Ende des Venturi-Rohrs ist bevorzugt mit einem Schalldämpfer versehen.

Die Steuereinrichtung in Form des Computers weist bevorzugt eine Fernbedienung, kombiniert mit einem Empfänger auf.

Die Fernbedienung weist hierbei bevorzugt mit der Steuereinrichtung Sende/Empfangsverbindungen zur Bedienung und zur Informationsabfrage auf.

Der Computer weist weiterhin ein Computerprogramm für einen Dialog zur Optimierung der Atemhilfe und für Autopilotfunktion auf.

Bei einer vorteilhaften Weitergestaltung des erfindungsgemäßen Verfahrens kann zur Sauerstoffdosierung in den Beatmungsgasen bei Konzentrationen des O₂ von mehr als 50% mittels einer Blende und Steuer/Kontrolleinrichtungen die Einsaugung der Raumluft ins Venturi-Rohr dosiert begrenzt werden, und das Verhältnis der Öffnungszeit zweier Magnetventile während der inspiratorischen Phase beliebig geregelt werden.

Weiterhin kann impulsweise z. B. Bidistillatwasser mittels einer Einrichtung aus einem Blitzverdampfer, einer Pumpe, einem Behälter und einem Computer computergesteuert nach Menge und Temperatur im Hochdruckkanal verdampft und in die Injektordüse verabreicht werden.

Zur Linderung der Geräusche im Venturi-Rohr kann am distalen Ende des Rohres ein Filter-Befeuchter oder eine künstliche Nase angeschlossen werden.

Weiterhin kann zur Dosierung der Beatmungsdrücke und Volumina die Querschnittsweite einer Variodüse im Venturi-Rohr während der Inspiration proportional zu der Flowsenkung in den Atemwegen des Patientens verengt werden; der Ausgangsdruck im Venturi-Rohr während der Inspiration um eine Differenz zum steigenden Atemwegdruck mehr als 0 mbar aufrechterhalten werden; und der positive endexpiratorische Druck (PEEP) mit einer Kombination von Verengung der Variodüse und einem Gegenfluß aus der Injektordüse eingestellt werden.

Das erfindungsgemäße Beatmungsgerät zeichnet sich durch eine Vielzahl von bisher in diesem Umfang nicht realisierbaren bzw. kombinierbaren Funktionen aus, nämlich unter anderem:

Es kann im Modus "Autopilot" automatisch die jeweils optimalen Beatmungsparameter aufrecht erhalten. Es kann Diagnosen, Symptome, Laborwerte und andere Informationen berücksichtigen; einen Dialog mit dem behandelnden Arzt über eine Optimierung der Beatmung durchführen; es kann mit einer Fernbedienung versehen werden; es ist nicht notwendig, daß es sterilisiert wird; es ist mit EKG synchronisierbar; es ermöglicht die Diagnose einiger pathologischer Prozesse in der Lunge, wie z. B. eines Bronochspasmus, einer Bronchusobstruktion sowie einer Verschleimung der Lunge; es ermöglicht kontinuierliche intratracheale Instillation von Medikamenten, Dampfbefeuchtung und Wärmung der Beatmungsgase; schaltet automatisch von spontaner Atmung auf assistierte, synchronisierte sowie kontrollierte Beatmung um und macht dies auch umgekehrt; es signalisiert die Notwendigkeit des Austausches der künstlichen Nase; es ermöglicht Beatmungsformen mit Hochfrequenz, Schaukel- und Pendelfrequenzen und Mischformen daraus; es ermöglicht variable Atemzugsvolumina bei gleichbleibendem Atemminutenvolumen; es ermöglicht die Verwendung von PEEP und CPAP während bestimmter Zeiträume der Exspiration, pulsierenden PEEP und CPAP und die Regulierung des Widerstandes während der Inspiration bei Spontanatmung; es ermöglicht die Verwendung aller handelsüblichen Inhalatoren und Vernebler; und es ermöglicht eine erhebliche Sauerstoffeinsparung und die Verwendung von Sauerstoffgeneratoren.

Weitere Einzelheiten, Aspekte und Vorteile der Erfindung ergeben sich aus der nachfolgenden Beschreibung unter Bezugnahme auf die Zeichnung.

Es zeigt:
Fig. 1 ein schematisches Blockdiagramm einer Ausführungsform des erfindungsgemäßen Beatmungsgerätes;
Fig. 2 und Fig. 3 einige Beispiele für Druckkurven im Trachealtubus, die das erfindungsgemäße Beatmungsgerät darstellen kann;
Fig. 4 einige Beispiele der Verhältnisse zwischen dem Druck (Fluß) in der Injektordüse und der Querschnittsweite der Variodüse bei verschiedenen Flowmustern und Atemwegedrücken; und
Fig. 5 einen elektropneumatischen Umwandler und die Verhältnisse zwischen elektronischem Signal, das den Umwandler steuert und der Druckkurve am pneumatischen Ausgang des Umwandlers.

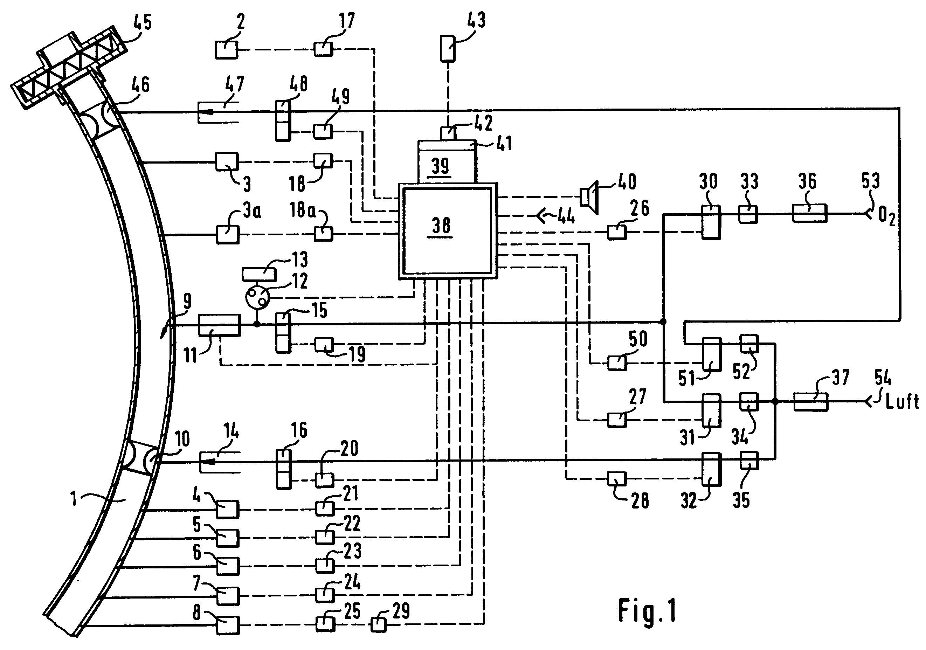

Fig. 1 zeigt eine Ausführungsform des erfindunggemäßen Beatmungsgerätes mit einem Beatmungskanal 1 in Form eines sogenannten Venturi-Rohrs mit einem zur Umgebung hin offenen distalen, d.h. bezogen auf den Patienten körperfernen oder vom Körper abgewandten Ende mit einem Nasen-Schalldämpfer 45 und einem proximalen Ende, sowie mit Sensoren 2 bis 8, einer Injektordüse 9, einer Variodüse 10, einem Blitzverdampfer-Gaserwärmer 11, einer Wasserpumpe 12, einem Behälter 13, Mikroventuri-Rohren 14 und 47, einem elektropneumatischen Umwandler 15, mikroelektropneumatischen Umwandlern 16 und 48, Verstärkern 17 bis 28, sowie 49 und 50, einem Tonfrequenzfilter 29, Magnetventilen 30 bis 32 und 51, Druckminderern 33 bis 35 und 52, Filtern 36 und 37, einer zentralen Steuervorrichtung in Form eines Computers 38, einem Bildschirm 39, einem Alarmsystem 40, einer Bedienungstastatur 41, einem Fernbedienungssensor-Sender 42, einem Fernbedienungs-Empfänger 43, einem EKG-Eingang 44 und einer Varioblende 46. Die Sensoren 2 bis 8 umfassen Drucksensoren 3, 3a und 4, einen endexspiratorischem Sauerstoffkonzentrationssensor 5, einen Kohlendioxidsensor 6, einen Temperatursensor 7, einen Geräuschesensor 8 und einen Sauerstoffsättigungsensor 2. Weiterhin vorgesehen ist ein Tonfrequenzfilter 29.

In Fig. 1 sind mit gestrichelten Linien elektrische oder elektronische Verbindungen dargestellt und mit durchgezogenen Linien pneumatische Verbindungen.

Konkreter Aufbau bzw. Typ, genaue Betriebsparameter und Funktions- oder Wirkungsweisen des Venturi-Rohrs 1, der Sensoren 2 bis 8, der Injektordüse 9, der Variodüse 10, des Blitzverdampfer-Gaserwärmers 11, der Wasserpumpe 12, des Behälters 13, der Mikroventuri-Rohre 14 und 47, des elektropneumatischen Umwandlers 15, der mikroelektropneumatischen Umwandler 16 und 48, der Verstärker 17 bis 28, 49 und 50, des Tonfrequenzfilters 29, der Magnetventile 30 bis 32 und 51, der Druckminderer 33 bis 35 und 52, der Filter 36 und 37, des Computers 38, des Bildschirms 39, des Alarmsystems 40, der Bedienungstastatur 41, der Fernbedienungseinrichtung, des EKG-Eingangs 44, des Nasen-Schalldämpfers 45, der Varioblende 46 und des Tonfrequenzfilters 29 sind dem Fachmann auf diesem Gebiet entweder geläufig, oder es erfolgt in der nachfolgenden Beschreibung noch eine genauere Spezifikation und/oder Erläuterung.

Nachfolgend sollen - weiterhin unter Bezugnahme auf die Zeichnung - die Funktionen des erfindungsgemäßen Beatmungsgerätes näher erläutert werden.

Zunächst soll kurz auf die wesentlichen Funktionen der einzelnen Elemente oder Komponenten eingegangen werden:

Der Fluß (Druck) in der Injektordüse 9 bestimmt die Atemvolumina. Die Ausströmrichtung aus der Injektordüse ist hierbei im wesentlichen senkrecht zu einer Ebene, welche von der Querschnittsfläche der Variodüse 10 definiert wird. Weiterhin weist der Beatmungskanal 1 die aus Fig. 1 ersichtliche Krümmung derart auf, daß die Mündung der Injektordüse 9 in die Mitte des Querschnittsfläche der Variodüse zielt. Somit wird die Variodüse 10 in einer Richtung durchströmt, welche im wesentlichen parallel zur Ausströmrichtung der Injektordüse 9 liegt.

Die Variodüse 10 bestimmt die Einsaugskraft in der Venturi-Einheit, stabilisiert die Kraft bei steigendem Widerstand der Atemwege während der Inspiration, verhindert die Flußumkehr und ermöglicht somit volumenkonstante Beatmungsformen. Während der Expiration bestimmt sie auch die expiratorische Drücke und kann bei Messung der Compliance der Lunge völlig geschlossen werden.

Die Menge der eingesaugter Raumluft während der Inspiration wird mit Hilfe der Varioblende 46 begrenzt oder völlig verhindert. Die Variodüse 10 und die Varioblende 46 werden zur Erhöhung der Triggerempfindlichkeit geschlossen.

Nach den Ausgangssignalen des endexpiratorischen Sauerstoffkonzentrationssensors 5 werden die Einsaugkraft (Variodüse 10), die Menge der eingesaugten Raumluft (Varioblende 46) und die Verhältnisse der Öffnungszeit der Magnetventile 30 und 31 geregelt.

Die Querschnittsweite der Variodüse 10 bestimmt somit die Drücke bei Beatmung, verhindert die Flußumkehr im Venturi-Rohr 1 und kann bei Messung der Lungenresistance und Compliance völlig geschlossen werden.

Die inspiratorische Sauerstoffkonzentration kann auch zusätzlich mittels Änderung der Öffnungszeitverhältnisse während der Inspiration durch die Magnetventile 30 und 31 aufrechterhalten und nach der endexpiratorischen Sauerstoffkonzentration - gemessen vom Sensor 5 - eingestellt werden. Außerdem wird bei Beatmung mit erheblichen Sauerstoffkonzentrationen über 50% die Varioblende 46 benutzt werden, die bei Verengung die Menge der eingesaugten Raumluft während der Inspiration begrenzt oder völlig verhindert. Die Blende 46 wird mit Hilfe des Mikroventurirohres 47 und des computergesteuerten mikroelektropneumatischen Umwandlers 48 verengt oder erweitert.

Die inspiratorischen Beatmungsgase werden mit Hilfe einer computergesteuerten Einrichtung aus Wasserpumpe 12 und Blitzverdampfer 11 befeuchtet und erwärmt.

Die Funktionen der einzelnen Sensoren sind wie folgt: die Drucksensoren 3, 3a und 4 messen die Drücke, Druckdifferenzen (Volumina), Lungenresistance und Compliance und signalisieren die Notwendigkeit des Austausches des künstlichen Nasen-Schalldämpfers 45. Die endexspiratorische Sauerstoffkonzentration in den Beatmungsgasen wird mit Hilfe des Sensors 5 gemessen, die Kohlendioxidkonzentration mit Hilfe des Sensors 6. Die Temperatur der Gase wird mit dem Sensor 7 gemessen. Der Geräuschesensor 8 und der Tonfrequenzfilter 29 erkennen Geräusche, die für Spastik, Verschleimung der Lunge und andere pathologische Prozesse in den Atemwegen kennzeichnend sind.

Alle Funktionen des Gerätes werden über den Computer 38 gesteuert. Ein beliebiger Abschnitt einer beliebigen Atemkurve, dargestellt auf dem Bildschirm 39, kann mit Hilfe der Bedienungstastatur 41 oder der Fernbedienung 43 zur Optimierung der Beatmung ausgewählt und geändert werden. Informationen können auch in einer Entfernung vom Gerät mit Hilfe des Empfängers der Fernbedienung abgefragt werden. Dies kann auch vollautomatisch erfolgen im Modus "Autopilot". Außerdem gestatten verschiedene Programme die Durchführung eines Dialogs mit dem Beatmungsgerät zur Optimierung der Beatmung und Behandlung der respiratorischen Insuffizienz.

Nachfolgend erfolgt eine genauere Erläuterung der Arbeitsweise und der Funktionen des erfindungsgemäßen Beatmungsgerätes:

Der Flow im proximalen Ende des Venturi-Rohres ist von folgenden Faktoren abhängig:
a) Druck (Flußgeschwindigkeit) in der Injektordüse 9;
b) Querschnittsweite der variablen Verengung oder Variodüse 10; und
c) Compliance der Lunge bzw. Atemwege.

Da der Widerstand der Atemwege am Ende der Inspiration steigt, wird die Flußgeschwindigkeit in der Injektordüse 9 gesenkt und die Querschnittsweite der Variodüse 10 verengt. So kann der Flow mittels Druckveränderung in der Injektordüse 9 und Querschnittsweite der Verengung der Variodüse 10 (bei bekannter Compliance, die vorher gemessen wird) bestimmt werden.

Wichtig ist die Druckdifferenz während der Exspiration zwischen den Drucksensoren 3 und 3a, welche am distalen Ende des Venturi-Rohres 1 angeordnet sind, wodurch der Rechner oder Computer 38 das exspiratorische Volumen bestimmt. Da das erfindungsgemäße Beatmungsgerät keine Beatmungsschläuche, Ventile und potentiellen Undichtigkeitsstellen hat, ist das in- und exspiratorische Volumen immer gleich. Deshalb braucht das inspiratorische Volumen nicht gemessen werden.

Die Funktionen der variablen Verengung oder Variodüse 10 umfassen im wesentlichen:
a) Anpassung des Flows mittels Änderung der inspiratorischen Druckleistung des Venturi-Rohres 1;
b) Stabilisierung des Atemwegdruckes bei Flowänderung infolge von Complianceänderungen;
c) Dosierung des Widerstandes während der Exspiration und während der Inspiration bei Spontanatmung;
d) Vermeidung der Flußumkehr (Änderung der Richtung) im Venturi-Rohr 1 für den Fall, daß der Widerstand der Atemwege die inspiratorische Druckleistung des Venturi-Rohres 1 erreichen kann;
e) Einsparung des Druckgasverbrauchs;
f) Erhöhung der Triggerempfindlichkeit; und
g) Anpassung des Flows an Alter und Gewicht des Patienten.

Die Funktionen der Blende 46 lassen sich im wesentlichen wie folgt angeben:
a) Begrenzung der Raumlufteinsaugung und damit Erhöhung der FiO₂;
b) Messung der Parameter der Atemmechanik; und
c) endinspiratorische Pause.

Die Funktionen der einzelnen Sensoren sind wie folgt:

Der Sensor 2 wird an der Haut des Patienten befestigt und kontrolliert die Sättigung des Blutes mit Sauerstoff. Die Sensoren 3 und 3a kontrollieren die Druckdifferenz im Venturi-Rohr 1 während der Exspiration zur Berechnung des exspiratorischen Volumens. Außerdem mißt der Sensor 3 während der Exspiration den Druck (Widerstand) zwischen den Atemwegen und der Nase 45 und signalisiert bei Anstieg des Druckes die Notwendigkeit, die Nase auszutauschen. Der Sensor 4 kontrolliert während der Beatmung die Drücke in den Atemwegen. Die Sensoren 5 und 6 messen die Sauerstoff- und Kohlendioxidkonzentration am Ende der Exspiration und können an beliebiger Stelle des Venturi-Rohres 1 angeordnet werden (aber je näher zum Patient, desto präziser die Werte). Der Sensor 7 mißt die Temperatur der Gase während der Inspiration und muß möglichst weit von der Injektordüse entfernt sein, weil von der Injektordüse Gas mit erhöhter Temperatur eingeblasen wird, das sich mit der Raumluft mischt und in die Atemwege verabreicht wird. Der Geräuschesensor 8 (z.B. ein Mikrophon) sollte möglichst nahe am Patienten angeordnet werden, um Geräusche aus den Atemwegen zu empfangen.

Während der Exspiration mit PEEP (positiver endexpiratorischer Druck) wird der Widerstand der Ausatmung durch Verengung der Variodüse 10 und (bei Bedarf) zusätzlich durch Gegenfluß aus der Injektordüse 9 erreicht. Während der Exspiration ohne PEEP ist die Variodüse maximal geöffnet.

Die Flußgeschwindigkeit in der Injektordüse 9 ergibt sich als Funktion des elektropneumatischen Umwandlers 15 und der Steuereinrichtung bzw. des Computers 38, die bzw. der alle Werte der Sensoren berücksichtigt.

Die Querschnittsweite der Verengung 10 ergibt sich als Funktion des Mikroinjektors 14, des mikroelektropneumatischen Umwandlers 16 und der Steuereinrichtung bzw. des Computers 38, die bzw. der alle Werte der Sensoren berücksichtigt.

Mit Verengung des Querschnitts 10 steigt die Druckleistung des Venturi-Rohres 1. Mit Erhöhung der Flußgeschwindigkeit in der Injektordüse 9 steigt der Flow.

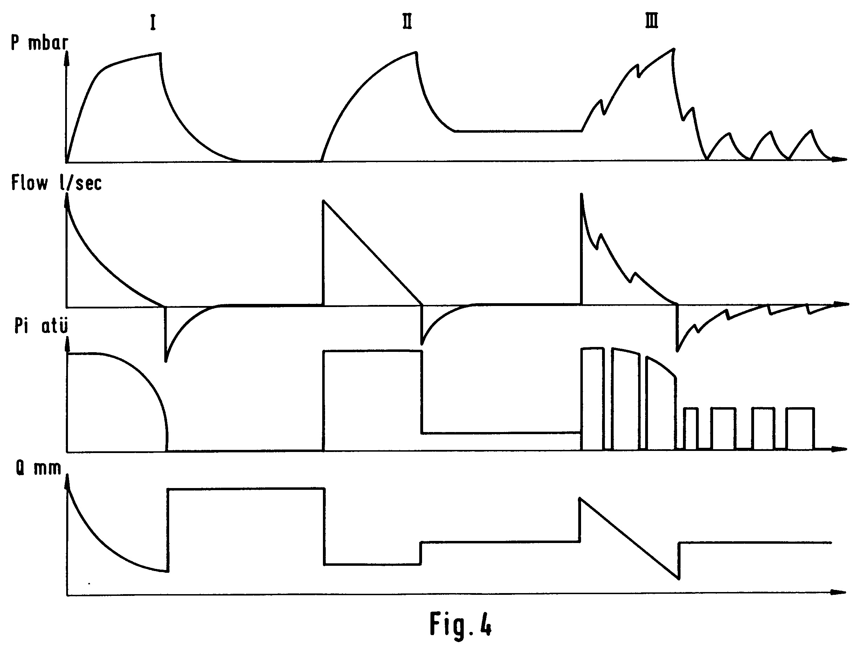

Änderungen des Querschnittes 10 des Venturi-Rohres 1 und der Flußgeschwindigkeit in der Injektordüse 9 gestatten es, beliebige Flowmuster darzustellen und den Flow während beliebiger Zeitabschnitte des Atemzyklusses zu senken, zu erhöhen oder konstant zu lassen, wie dies in Fig. 4 exemplarisch dargestellt ist.

Der passive Befeuchter/Schalldämpfer 45 vermindert die Gaswärmeverluste, die Gasfeuchtigkeitsverluste und reduziert die Geräusche des Venturi-Rohres 1.

Der aktive Blitz-Gaserwärmer 11 verdampft das mit entsprechender Geschwindigkeit mit Hilfe der Wasserpumpe 12 verabreichte Wasser, z. B. Bidestillatwasser und befeuchtet und erwärmt somit die inspiratorischen Gase.

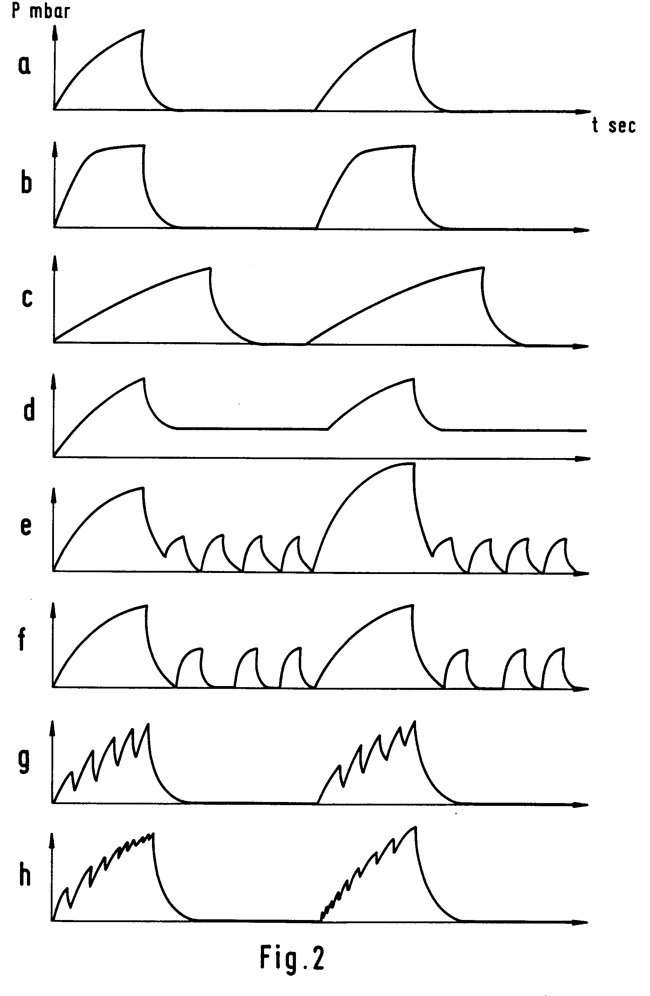

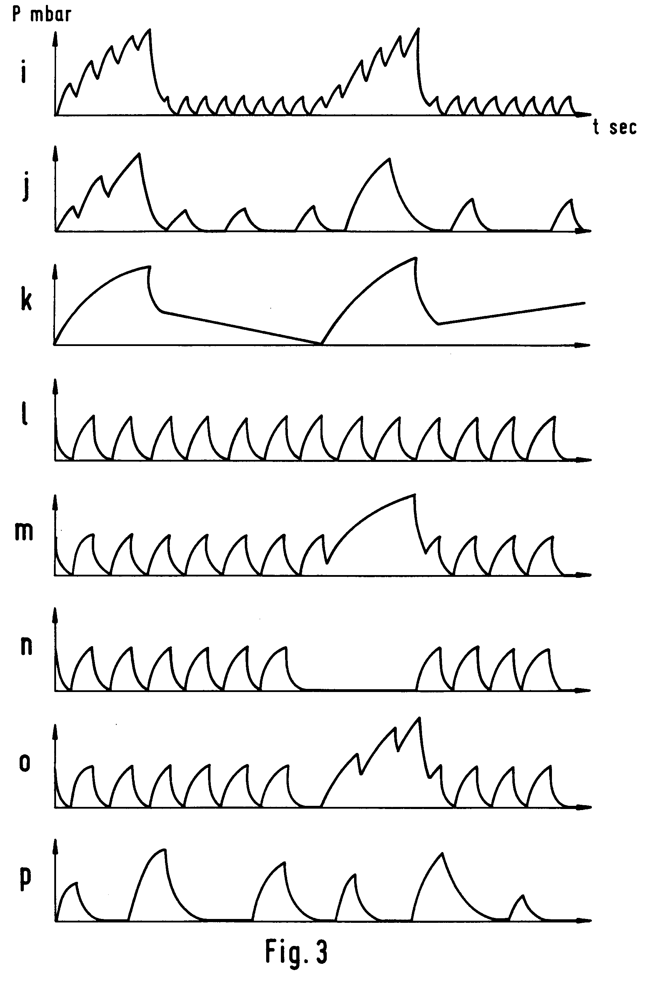

In Fig. 2 und 3 sind Beispiele von Atemwegedruckkurven während der Beatmung dargestellt:
a - volumenkontrollierte Beatmung;
b - inspiratorisches Plateau;
c - Inversed Ratio Ventilatio (IRV);
d - PEEP (positiver endexspiratorischer Druck);
e - pulsierender PEEP (Hochfrequenz-PEEP) und ein Seufzer;
f - PEEP mit schaukelnden hohen Frequenzen;
g - hochfrequenzmodulierte Inspiration;
h - schaukelfrequenzmodulierte Inspiration;
i - Mischform von üblicher Beatmung mit Hochfrequenzoszillationen;
j - wie i, aber mit Schaukelhochfrequenzen;
k - deszendierender und aszendierender PEEP;
l - HFB mit fixierter Frequenz;
m - KFB mit bradypnoischen Inspirationen;
m - HFB mit Pausen;
o - HFB mit bradypnoischer, HF-modulierten Inspirationen; und
p - Pendelfrequenzbeatmung mit instabilen Atemzugvolumina bei gleichbleibendem Minutenvolumen.

Die Graphik in Fig. 4 zeigt einige Beispiele der Verhältnisse zwischen dem Druck (Fluß) in der Injektordüse 9 und der Querschnittsweite der Variodüse 10 bei verschiedenen Flowmustern und Atemwegedrücken. Hierbei sind:
- P mbar: : der Druck in den Atemwegen,
- Flow 1/sec: : Flow in den Atemwegen,
- Pi atü: : der Druck in der Injektordüse 9, und
- Q mm: : Querschnittsweite der Variodüse 10 in mm.

Die P/Flow/Pi/Q-Verhältnisse sind in Fig. 4 dargestellt als bei I bei üblicher volumenkontrollierter Beatmung; bei II mit PEEP (positivem endexspiratorischem Druck); und bei III als Mischformen mit Hochfrequenzoszillationen während des ganzen Atemzyklusses.

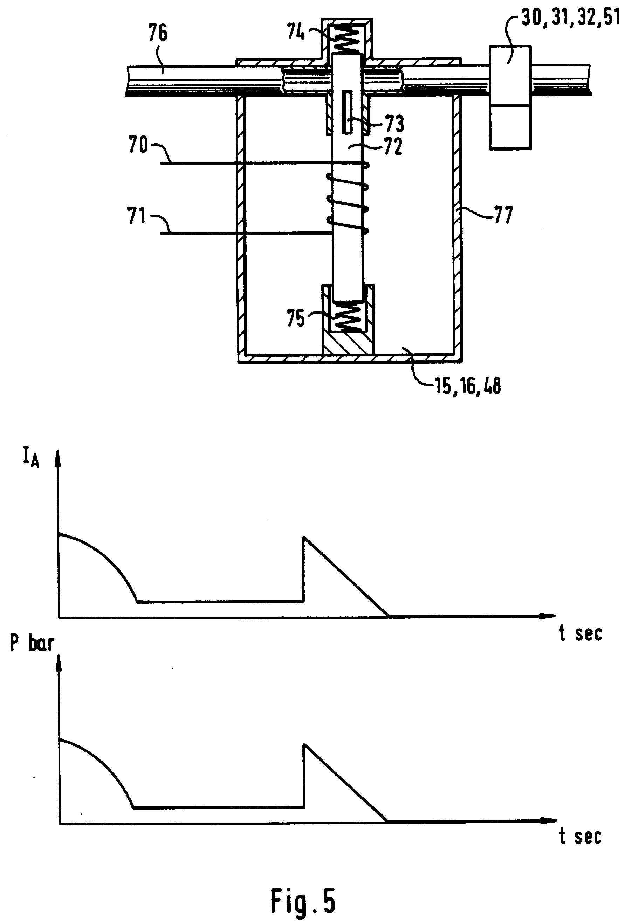

Unter Bezug auf Fig. 5 erfolgt nun eine Beschreibung der elektropneumatischen Umwandler 15, 16 und 48.

Die elektropneumatischen Umwandler 15, 16 und 48 sind Einrichtungen, die an den Hochdruckkanälen angeordnet sind und zur Umwandlung beliebiger elektrischer Kurven bzw. Signalverläufe, die im Computer 38 generiert werden, in eine entsprechende Druckkurve geeignet sind. In dem in Fig. 5 unteren Diagramm ist eine Strom/Druckkurve dargestellt, wobei J_{A} der Strom in der Spule des Umwandlers ist und Pbar der Druck am Ausgang des Umwandlers ist.

Der elektropneumatische Umwandler 15, 16 bzw. 48 besteht gemäß der Darstellung von Fig. 5 aus einer Magnetspule 70, 71, in deren Magnetfeld ein Stäbchen 72 mit einer seitlichen Öffnung 73 leicht bewegbar ist, und zwar variabel nach Frequenz, Amplitude und zeitlichem Intervall. Um die Trägheit des Stäbchens 72 zu vermindern, ist das Stäbchen 72 aus einem leichten Werkstoff gemacht und oben und unten mit Federn 74 und 75 aufgehängt, oder auf einem gespannten Seidenfaden aufgehängt. Die seitliche Öffnung 73 öffnet bei Bewegung mehr oder weniger den Hochdruckkanal 76, der quer über der Magnetspulenkapsel 77 liegt. Ein Elektromagnetventil 30, 31, 32, oder 51 vor oder nach dem Stäbchen 72 schließt bei Bedarf den Fluß im Hochdruckkanal 76, um einen positiven endexspiratorischen Druck in den Atemwegen des Patienten zu verhindern.

Insoweit zusammenfassend wird unter anderem bevorzugt, daß
zur Oxygenationskontrolle mit Hilfe des Sensors 5 die endexspiratorische Sauerstoffkonzentration im Beatmungskanal gemessen wird;
zur Sauerstoffdosierung in den Beatmungsgasen bei Konzentrationen des O₂ von mehr als 50% mittels der Varioblende 46 die Einsaugung der Raumluft ins Venturi-Rohr dosiert begrenzt wird, und das Verhältnis der Öffnungszeit der Magnetventile 30 und 31 während der inspiratorischen Phase beliebig geregelt wird; und
zur Dosierung der Beatmungsdrücke und Volumina
   - die Querschnittsweite der Variodüse 10 im Venturi-Rohr 1 während der Inspiration proportional zu der Flowsenkung verengt wird;
   - der Ausgangsdruck im Venturi-Rohr 1 während der Inspiration um eine Differenz zum steigenden Atemwegedruck mehr als 0 mbar aufrechterhalten wird; und
   - der positive endexspiratorische Druck (PEEP) mit Verengung der Variodüse 10 oder zusätzlich mit einem Gegenfluß aus der Injektordüse 9 eingestellt wird.

## Patentansprüche

1. Beatmungsgerät mit:
einem mit dem Patienten verbindbaren Beatmungskanal (1) mit einem proximalen und einem distalen Ende;
Hochdruckgasanschlüssen (53, 54) für Beatmungsgase;
einer Steuereinrichtung (38) zum Festlegen von Beatmungsparametern; und
einer Einrichtung (15, 19) zur Dosierung der Beatmungsgase,
wobei der Beatmungskanal (1) in Form eines Venturi-Rohres eine Variodüse (10) mit einstellbarem Querschnitt flußabwärts einer Injektordüse (9), eine Varioblende (46) mit einstellbarem Querschnitt flußaufwärts der Injektordüse (9), welche die Menge der eingesaugten Raumluft dosiert begrenzt aufweist, dadurch gekennzeichnet, daß der Beatmungskanal (1) auch einen endexpiratorischen Sauerstoffkonzentrationssensor (5) zur Oxygenationskontrolle aufweist, wobei die Ausgangssignale des Sauerstoffkonzentrationssensors (5) zur Regelung der variablen Querschnitte der Varioblende (46) und zur Einstellung des Querschnittes der Variodüse (10) verwendet werden.

2. Beatmungsgerät nach Anspruch 1, dadurch gekennzeichnet, daß die Variodüse (10) einen starren äußeren Zylinder und einen inneren elastischen und zur Mitte bewegbaren Zylinder mit einem dazwischen liegenden Raum und ein Mikroventuri-Rohr (14), das am äußeren starren Zylinder angeordnet ist, aufweist.

3. Beatmungsgerät nach Anspruch 1 oder 2, dadurch gekennzeichnet, daß das Venturi-Rohr (1) derart gekrümmt ist, daß der innere Zylinder der Variodüse (10) im wesentlichen parallel zu der Ausströmrichtung der Injektordüse (9) liegt.

4. Beatmungsgerät nach Anspruch 1, 2 oder 3, dadurch gekennzeichnet, daß die Variodüse (10) mit einer Steuereinrichtung zum Einbringen eines Druckes in den Raum zwischen den Zylindern versehen ist, welche das Mikroventuri-Rohr (14), einen mikroelektropneumatischen Umwandler (16) und einen Computer (38) aufweist.

5. Beatmungsgerät nach einem der vorhergehenden Ansprüche, dadurch gekennzeichnet, daß der Raum zwischen dem äußeren und dem inneren Zylinder der Variodüse (10) über das Mikroventuri-Rohr (14) mit der Umgebung in Verbindung steht.

6. Beatmungsgerät nach einem der vorhergehenden Ansprüche, dadurch gekennzeichnet, daß der Raum zwischen dem äußeren und inneren Zylinder der Variodüse (10) über einen mikroelektropneumatischen Wandler (16) mit dem Hochdruckgasanschluß (54) verbunden ist.

7. Beatmungsgerät nach Anspruch 1, dadurch gekennzeichnet, daß die Dosiereinrichtung (15) einen elektropneumatischen Umwandler für hohe Drücke aufweist.

8. Beatmungsgerät nach einem der vorhergehenden Ansprüche, dadurch gekennzeichnet, daß die Varioblende (46) einen starren äußeren Zylinder, einen inneren elastischen und zur Mitte bewegbaren Zylinder und einen dazwischen liegenden Raum aufweist, wobei die Verengungen des Innenlumens auch völlig verschließbar ist, sowie ein Mikroventuri-Rohr (47) aufweist, das am äußeren starren Zylinder angeordnet ist.

9. Beatmungsgerät nach Anspruch 8, dadurch gekennzeichnet, daß die Varioblende (46) mit einer Steuereinrichtung mit dem Mikroventuri-Rohr (47), einem mikroelektropneumatischen Umwandler (48) und dem Computer (38) zum Einbringen eines Druckes in den Raum zwischen den Zylindern verbunden ist.

10. Beatmungsgerät nach einem der vorhergehenden Ansprüche, dadurch gekennzeichnet, daß das Venturi-Rohr (1) einen Geräuschesensor (8) aufweist.

11. Beatmungsgerät nach einem der vorhergehenden Ansprüche, dadurch gekennzeichnet, daß es im Hochdruckkanal oberhalb der Injektordüse (9) einen Blitzwasserverdampfer (11) aufweist.

12. Beatmungsgerät nach Anspruch 11, dadurch gekennzeichnet, daß der Blitzwasserverdampfer (11) Fluidverbindungen zu einer Wasserpumpe (12), pneumatische Verbindungen zur Injektordüse (9) und zum elektropneumatischen Umwandler (15), sowie elektronische zum Computer (38) aufweist.

13. Beatmungsgerät nach einem der vorhergehenden Ansprüche, dadurch gekennzeichnet, daß das distale Ende des Venturi-Rohrs (1) einen Schalldämpfer (45) aufweist.

14. Beatmungsgerät nach einem der vorhergehenden Ansprüche, dadurch gekennzeichnet, daß die Steuereinrichtung in Form des Computers (38) eine Fernbedienung, kombiniert mit einem Empfänger (43), aufweist.

15. Beatmungsgerät nach Anspruch 14, dadurch gekennzeichnet, daß die Fernbedienung (43) mit der Steuereinrichtung (38) Sende/Empfangsverbindungen zur Bedienung und zur Informationsabfrage aufweist.

16. Beatmungsgerät nach Anspruch 14, dadurch gekennzeichnet, daß der Computer (38) ein Computerprogramm für einen Dialog zur Optimierung der Atemhilfe und für Autopilotfunktion aufweist.

17. Verfahren zum Betrieb eines Beatmungsgerätes, insbesondere eines Beatmungsgerätes nach einem der Ansprüche 1 bis 16, wobei zur Dosierung des Sauerstoffes in den Beatmungsgasen die folgenden Schritte unternommen werden:
Anordnen einer Variodüse (10) in einem Beatmungskanal (1) in Form eines Venturi-Rohres mit einstellbarem Querschnitt flußabwärts einer Injektordüse (9), einer Varioblende (46) mit einstellbarem Querschnitt flußaufwärts der Injektordüse (9), welche die Menge der eingesaugten Raumluft dosiert begrenzt und eines endexpiratorischen Sauerstoffkonzentrationssensors (5); Messung der endexpiratorischen Sauerstoffkonzentration im Beatmungskanal (1) zwecks Oxigenationskontrolle mit Hilfe des Sauerstoffkonzentrationssensors (5); und
Verwenden der Ausgangssignale des Sauerstoffkonzentrationssensors (5) zur Regelung der variablen Querschnitte der Varioblende (46) und zur Einstellung des Querschnittes der Variodüse (10).

18. Verfahren nach Anspruch 17, wobei zur Sauerstoffdosierung in den Beatmungsgasen bei Konzentrationen des O₂ von mehr als 50 % mittels der Blende (46) und Steuer/Kontrolleinrichtungen (47, 48, 49, 50, 51, 52, 38) die Einsaugung der Raumluft ins Venturi-Rohr dosiert begrenzt wird, und das Verhältnis der Öffnungszeit zweier Magnetventile (30, 31) während der inspiratorischen Phase beliebig geregelt wird.

19. Verfahren nach Anspruch 17 oder 18, wobei impulsweise z. B. Bidistillatwasser mittels einer Einrichtung aus einem Blitzverdampfer (11), einer Pumpe (12), einem Behälter (13) und einem Computer (38) computergesteuert nach Menge und Temperatur im Hochdruckkanal verdampft und in die Injektordüse (9) verabreicht wird.

20. Verfahren nach einem der Ansprüche 17 bis 19, wobei zur Linderung der Geräusche im Venturi-Rohr (1) am distalen Ende des Rohres (1) ein Filter-Befeuchter oder eine künstliche Nase (45) angeschlossen wird.

21. Verfahren nach einem der Ansprüche 17 bis 20, wobei zur Dosierung der Beatmungsdrücke und Volumina
- die Querschnittsweite einer Variodüse (10) im Venturi-Rohr (1) während der Inspiration proportional zu der Flowsenkung in den Atemwegen des Patientens verengt wird;
- der Ausgangsdruck im Venturi-Rohr (1) während der Inspiration um eine Differenz zum steigenden Atemwegdruck mehr als 0 mbar aufrechterhalten wird; und
- der positive endexpiratorische Druck (PEEP) mit einer Kombination von Verengung der Variodüse (10) und einem Gegenfluß aus der Injektordüse (9) eingestellt wird.

## Claims

1. A respirator with:
a respiration channel (1) which is adapted to be connected to the patient and which has a proximal and a distal end;
high-pressure gas connections (53, 54) for respiration gases;
a control device (38) for determination of respiration parameters; and
a device (15, 19) for metering the respiration gases,
wherein the respiration channel (1) in the form of a venturi tube has a variable nozzle (10) of adjustable cross-section downstream of an injector nozzle (9), a variable screen (46) of adjustable cross-section upstream of the injector nozzle (9), which with metering limits the quantity of intaken atmospheric air, characterised in that the respiration channel (1) also comprises an end expiratory oxygen concentration sensor (5) for oxygenation control, the output signals of the oxygen concentration sensor (5) being used to control the variable cross-sections of the variable screen (46) and for adjusting the cross-section of the variable nozzle (10).

2. A respirator according to claim 1, characterised in that the variable nozzle (10) comprises a rigid outer cylinder and an inner elastic cylinder movable relatively to the centre and having an intermediate pace and a microventuri tube (14) which is disposed on the outer rigid cylinder.

3. A respirator according to claim 1 or 2, characterised in that the venturi tube (1) is so curved that the inner cylinder of the variable nozzle (10) lies substantially parallel to the outflow direction of the injector nozzle (9).

4. A respirator according to claim 1, 2 or 3, characterised in that the variable nozzle (10) is provided with a control device for introducing a pressure into the space between the cylinders, which device comprises the microventuri tube (14), a microelectropneumatic transducer (16) and a computer (38).

5. A respirator according to any one of the preceding claims, characterised in that the space between the outer and inner cylinders of the variable nozzle (10) communicates with the atmosphere via the microventuri tube (14).

6. A respirator according to any one of the preceding claims, characterised in that the space between the outer and inner cylinders of the variable nozzle (10) is connected to the high-pressure gas connection via a microelectropneumatic transducer (16).

7. A respirator according to claim 1, characterised in that the metering device (15) comprises an electropneumatic transducer for high pressures.

8. A respirator according to any one of the preceding claims, characterised in that the variable screen (46) comprises a rigid outer cylinder, an inner elastic cylinder movable relatively to the centre and an intermediate space, the constrictions of the inner gap also being completely closable, and a microventuri tube (47) disposed on the outer rigid cylinder.

9. A respirator according to claim 8, characterised in that the variable screen (46) is connected to a control device with the micro-venturi tube (47), a microelectropneumatic transducer (48) and the computer (38) for introducing a pressure into the space between the cylinders.

10. A respirator according to any one of the preceding claims, characterised in that the venturi tube (1) has a noise sensor (8).

11. A respirator according to any one of the preceding claims, characterised in that it has a flash water evaporator (11) in the high-pressure channel above the injector nozzle (9).

12. A respirator according to claim 11, characterised in that the flash water evaporator (11) comprises fluid connections to a water pump (12), pneumatic connections to the injector nozzle (9) and to the electropneumatic transducer (15), and electronic connections to the computer (38).

13. A respirator according to any one of the preceding claims, characterised in that the distal end of the venturi tube (1) has a silencer (45).

14. A respirator according to any one of the preceding claims, characterised in that the control device in the form of a computer (38) has a remote operation combined with a receiver (43).

15. A respirator according to claim 14, characterised in that the remote operation (43) has transmission/reception connections to the control device (38) for operation and information interrogation.

16. A respirator according to claim 14, characterised in that the computer (38) has a computer program for a dialogue for optimising the respiratory assistance and for autopilot function.

17. A method of operating a respirator, more particularly a respirator according to any one of claims 1 to 16, comprising the following steps for metering the oxygen in the respiration gases:
provision of a variable nozzle (10) in a respiration channel (1) in the form of a venturi tube of adjustable cross-section downstream of an injector nozzle (9), a variable screen (46) of adjustable cross-section upstream of the injector nozzle (9), which with metering limits the quantity of intaken atmospheric air, and the provision of an end expiratory oxygen concentration sensor (5);
measuring the end expiratory oxygen concentration in the respiration channel (1) for oxygenation control by means of the oxygen concentration sensor (5); and
use of the output signals of the oxygen concentration sensor (5) to control the variable cross-sections of the variable screen (46) and for adjusting the cross-section of the variable nozzle (10).

18. A method according to claim 17, wherein for oxygen metering in the respiration gases in the case of O₂ concentrations of more than 50% the intake of atmospheric air into the venturi tube is limited with metering by means of the screen (46) and control/monitoring devices (47, 48, 49, 50, 51, 52, 38), and the ratio of the opening times of two solenoid valves (30, 31) during the inspiration phase is arbitrarily controlled.

19. A method according to claim 17 or 18, wherein bidistillate water, for example, is evaporated, by means of a device consisting of a flash evaporator (11), a pump (12), a container (13), and a computer (38), being computer-controlled as to quantity and temperature in the high-pressure channel, and is dispensed into the injector nozzle (9).

20. A method according to any one of claims 17 to 19, wherein to reduce the noise in the venturi tube (1) a filter-moistener or an artificial nose (45) is connected at the distal end of the tube (1).

21. A method according to any one of claims 17 to 20, wherein to meter the respiration pressures and volumes
- the cross-sectional width of a variable nozzle (10) in the venturi tube (1) is constricted during inspiration in proportion to the flow reduction in the patient's respiratory tracts;
- the output pressure in the venturi tube (1) is maintained during inspiration around a difference from the increasing respiratory tract pressure of more than 0 mbar; and
- the positive end expiratory pressure (PEEP) is adjusted by a combination of constriction of the variable nozzle (10) and a counter-current flow from the injector nozzle (9).

## Revendications

1. Appareil de respiration comportant:
un conduit de respiration (1) pouvant être branché sur le patient et présentant une extrémité proximale et une extrémité distale;
des raccords de gaz à haute pression (53, 54) pour des gaz de respiration;
un dispositif de commande (38) destiné à fixer des paramètres de respiration; et
un dispositif (15, 19) destiné à doser les gaz de respiration,
le conduit de respiration (1), qui se présente sous forme d'un tube de Venturi, comportant, en aval d'une buse d'injection (9), une buse à ouverture variable (< 10) de section transversale ajustable et, en amont de la buse d'injection (9), un diaphragme à ouverture variable (46) de section transversale ajustable, qui limite en le dosant le débit de l'air ambiant aspiré, caractérisé en ce que le conduit de respiration (1) comporte également un capteur de concentration en oxygène en fin d'expiration (5) pour le contrôle de l'oxygénation, les signaux de sortie du capteur de concentration en oxygène (5) étant alors utilisés pour le réglage de la section transversale variable du diaphragme à ouverture variable (46) et pour l'ajustement de la section transversale de la buse à ouverture variable (10).

2. Appareil de respiration selon la revendication 1, caractérisé en ce que la buse à ouverture variable (10) comporte un cylindre rigide extérieur et un cylindre intérieur élastique et déplaçable vers le centre, avec un espace situé entre eux, ainsi qu'un microtube de Venturi (14) qui est disposé sur le cylindre extérieur rigide.

3. Appareil de respiration selon la revendication 1 ou 2, caractérisé en ce que le tube de Venturi (1) est incurvé d'une façon telle, que le cylindre intérieur de la buse à ouverture variable (10) soit dans une position sensiblement parallèle à la direction de diffusion de la buse d'injection (9).

4. Appareil de respiration selon la revendication 1, 2 ou 3, caractérisé en ce que la buse à ouverture variable (10) est munie d'un dispositif de commande destiné à appliquer une pression dans l'espace formé entre les cylindres, dispositif de commande qui comporte le microtube de Venturi (14), un convertisseur microélectropneumatique (16) et un ordinateur (38).

5. Appareil de respiration selon l'une des revendications précédentes, caractérisé en ce que l'espace entre le cylindre extérieur et le cylindre intérieur de la buse à ouverture variable (10) communique avec l'environnement au travers du microtube de Venturi (14).

6. Appareil de respiration selon l'une des revendications précédentes, caractérisé en ce que l'espace entre le cylindre extérieur et le cylindre intérieur de la buse à ouverture variable (10) est relié, par l'intermédiaire d'un convertisseur microélectropneumatique (16), au raccord de gaz à haute pression (54).

7. Appareil de respiration selon la revendication 1, caractérisé en ce que le dispositif de dosage (15) comporte un convertisseur électropneumatique pour de hautes pressions.

8. Appareil de respiration selon l'une des revendications précédentes, caractérisé en ce que le diaphragme à ouverture variable (46) comporte un cylindre rigide extérieur, un cylindre intérieur élastique et déplaçable vers le centre et un espace situé entre ces deux cylindres, le rétrécissement de la lumière intérieure pouvant aussi être complètement obturé, ainsi qu'un microtube de Venturi (47) qui est disposé sur le cylindre extérieur rigide.

9. Appareil de respiration selon la revendication 8, caractérisé en ce que le diaphragme à ouverture variable (46) est relié à un dispositif de commande comportant le microtube de Venturi (47), un convertisseur microélectropneumatique (48) et l'ordinateur (38), pour l'application d'une pression dans l'espace formé entre les cylindres.

10. Appareil de respiration selon l'une des revendications précédentes, caractérisé en ce que le tube de Venturi (1) comporte un capteur de bruit (8).

11. Appareil de respiration selon l'une des revendications précédentes, caractérisé en ce qu'il comporte, dans le conduit haute pression et au-dessus de la buse d'injection (9), un évaporateur ultrarapide d'eau (11).

12. Appareil de respiration selon la revendication 11, caractérisé en ce que l'évaporateur ultrarapide d'eau (11) comporte des raccordements pour fluide le reliant à une pompe à eau (12), des raccordements pneumatiques le reliant à la buse d'injection (9) et au convertisseur électropneumatique (15), ainsi que des raccordements électroniques le reliant à l'ordinateur (38).

13. Appareil de respiration selon l'une des revendications précédentes, caractérisé en ce que l'extrémité distale du tube de Venturi (1) comporte un amortisseur de bruit (45).

14. Appareil de respiration selon l'une des revendications précédentes, caractérisé en ce que le dispositif de commande, sous forme de l'ordinateur (38), comporte une télécommande combinée à un récepteur (43).

15. Appareil de respiration selon la revendication 14, caractérisé en ce que la télécommande (43) présente des liaisons d'émission/réception avec le dispositif de commande (38), en vue de la commande et de la lecture d'informations.

16. Appareil de respiration selon la revendication 14, caractérisé en ce que l'ordinateur (38) possède un programme informatique pour assurer un dialogue, en vue d'optimiser l'assistance respiratoire, et pour assurer une fonction d'autopilotage.

17. Procédé d'exploitation d'un appareil de respiration, en particulier d'un appareil de respiration selon l'une des revendications 1 a 16, selon lequel, pour le dosage de l'oxygène dans les gaz de respiration, les étapes suivantes sont entreprises:
disposition, dans un conduit de respiration (1) se présentant sous la forme d'un tube de Venturi, d'une buse à ouverture variable (10) de section transversale ajustable, en aval d'une buse d'injection (9), et d'un diaphragme à ouverture variable (46) de section transversale ajustable en amont de la buse d'injection (9), lequel diaphragme limite en le dosant le débit de l'air ambiant aspiré, ainsi que d'un capteur de concentration en oxygène en fin d'expiration (5);
mesure de la concentration en oxygène, en fin d'expiration, dans le conduit de respiration (1) en vue d'un contrôle d'oxygénation, à l'aide du capteur de concentration en oxygène (5); et
utilisation des signaux de sortie du capteur de concentration en oxygène (5) pour le réglage de la section transversale variable du diaphragme à ouverture variable (46) et pour l'ajustement de la section transversale de la buse à ouverture variable (10).

18. Procédé selon la revendication 17, selon lequel, pour le dosage de l'oxygène dans les gaz de respiration à des concentrations en O₂ de plus de 50%, l'aspiration de l'air ambiant dans le tube de Venturi est limitée, sous dosage, au moyen du diaphragme (46) et des dispositifs de commande/contrôle (47, 48, 49, 50, 51, 52, 38), et le rapport des temps d'ouverture de deux électrovannes (30, 31), pendant la phase d'inspiration, est réglé à la valeur souhaitée.

19. Procédé selon la revendication 17 ou 18, selon lequel, sur un mode impulsionnel, de l'eau résultant, par exemple, d'une bidistillation est évaporée dans le conduit haute pression, sous commande informatisée, en fonction du débit et de la température, au moyen d'un dispositif constitué d'un évaporateur ultrarapide (11), d'une pompe (12), d'un récipient (13) et d'un ordinateur (38), et est administrée dans la buse d'injection (9).

20. Procédé selon l'une des revendications 17 à 19, selon lequel, pour atténuer les bruits dans le tube de Venturi (1), un filtre-humidificateur ou un nez artificiel (45) est raccordé à l'extrémité distale du tube (1).

21. Procédé selon l'une des revendications 17 à 20, selon lequel, pour le dosage des pressions de respiration et des volumes
- l'ampleur de la section transversale d'une buse à ouverture variable (10) dans le tube de Venturi (1) est, pendant l'inspiration, réduite par rétrécissement, proportionnellement à la diminution du débit dans les voies respiratoires du patient;
- la pression de sortie dans le tube de Venturi (1) est maintenue, pendant l'inspiration, à une différence de plus de 0 mbar par rapport à la pression croissante dans les voies respiratoires; et
- la pression positive en fin d'expiration (PEEP) est ajustée à l'aide d'une combinaison du rétrécissement de la buse à ouverture variable (10) et d'un flux à contre-courant provenant de la buse d'injection (9).
